# FASCICULE DE BREVET EUROPEEN

(11) **EP 2 732 848 B1**
(45) Date de publication et mention de la délivrance du brevet: **02.03.2022**
(21) Numéro de dépôt: 13173452.7
(22) Date de dépôt: 24.06.2013
(51) Int. Cl.: A61N 1/05

(54) **MICROSONDE IMPLANTABLE DANS LE RÉSEAU CORONARIEN PROFOND, COMPRENANT UNE PARTIE PROXIMALE DÉFORMABLE**
MIKROLEITER ZUR IMPLANTATION IN DIE TIEFEN HERZKRANZGEFÄSSE MIT EINEM VERFORMBAREN PROXIMALEN TEIL
MICROLEAD FOR IMPLANTATION IN THE DEEP CORONARY VESSELS COMPRISING A DEFORMABLE PROXIMAL PART

(30) Priorité: 14.11.2012 FR 1260840
(43) Date de publication de la demande: 21.05.2014
(73) Titulaire: Sorin CRM SAS, 92140 Clamart Cedex (FR)
(72) Inventeur: Régnier, Willy, 91160 Longjumeau (FR); Shan, Nicolas, 91260 Juvisy sur Orge (FR)
(74) Mandataire: Grünecker Patent- und Rechtsanwälte PartG mbB

(56) Documents cités:
- FR-A1- 2 801 510
- US-A1- 2008 183 257
- US-A1- 2010 010 605
- US-A1- 2010 114 017
- US-A1- 2011 071 585

## Description

L'invention concerne les "dispositifs médicaux implantables actifs" tels que définis par la Directive 90/385/CEE du 20 juin 1990 du Conseil des communautés européennes, notamment les implants permettant de surveiller en continu le rythme cardiaque et délivrer si nécessaire au cœur des impulsions électriques de stimulation ou de resynchronisation.

Elle concerne plus précisément les sondes de stimulation cardiaque destinées à être implantées dans le réseau coronarien du cœur pour permettre la stimulation d'une cavité gauche ou droite, ventricule ou oreillette. À la différence des cavités droites pour lesquelles il suffit d'implanter des sondes endocavitaires via le réseau veineux périphérique droit, la mise en place de sondes permanentes dans une cavité du cœur gauche impliquerait des risques opératoires importants, par exemple le risque de passage de bulles vers le réseau vasculaire cérébral situé en aval du ventricule gauche.

Pour cette raison, lorsque l'on veut stimuler une cavité gauche, on choisit le plus souvent d'introduire une sonde non pas dans la cavité à stimuler, mais dans le réseau coronarien, la sonde étant pourvue d'une électrode qui sera appliquée contre la paroi de l'épicarde au niveau du ventricule gauche ou de l'oreillette gauche, selon le cas. Ces sondes stimulent ainsi le muscle cardiaque via une ou plusieurs électrodes ponctuelles dont la position est fonction de la trajectoire prédéfinie de la veine canulée.

Une sonde de ce type est par exemple le modèle *Situs LV*, commercialisé par Sorin CRM (Clamart, France) et décrit dans le EP 0 993 840 A1 (ELA Médical).

L'introduction d'une telle sonde se fait par le sinus coronaire, depuis son débouché dans l'oreillette droite. La sonde est ensuite poussée et orientée le long du réseau des veines coronaires jusqu'au site choisi. Cette intervention est très délicate compte tenu des particularités du réseau veineux, avec notamment la diminution progressive de diamètre du conduit au fur et à mesure de la progression de la sonde dans la veine coronaire sélectionnée. Une fois la veine cible atteinte, le chirurgien recherche un site de stimulation satisfaisant, avec un bon contact électrique de l'électrode de stimulation contre le tissu de l'épicarde, ce contact devant être maintenu malgré les variations ou sollicitations diverses au cours du temps.

Une tendance récente en matière de sondes de stimulation du ventricule gauche est la réduction du diamètre de la partie implantable dans le réseau coronarien, jusqu'à un diamètre inférieur à 4 French (1,33 mm), voire jusqu'à moins de 2 French (0,33 mm).

La taille du corps de sonde est en effet un facteur directement lié aux capacités de guidage contrôlé de la sonde dans le réseau veineux coronarien, de manière à pouvoir y sélectionner des sites de stimulation particuliers situés dans certaines veines collatérales : le très faible diamètre extérieur de l'extrémité distale active de la sonde permet de canuler des veines très étroites du réseau coronarien, non exploitées à ce jour du fait de la taille excessive des sondes coronaires conventionnelles.

Une telle sonde est notamment décrite dans le EP 2 455 131 A1 (Sorin CRM). Elle comprend un corps de sonde avec une gaine creuse dans laquelle coulisse un microcâble présentant un diamètre de l'ordre de 0,5 à 2 French (0,17 à 0,66 mm), qui émerge sur une longueur de 1 à 200 mm au-delà du débouché du corps de sonde. Ce microcâble, qui constitue la partie active de la sonde, comporte une pluralité de parties dénudées formant une succession d'électrodes individuelles, constituant ensemble un réseau d'électrodes reliées en série permettant de multiplier les points de stimulation dans une zone profonde du réseau coronarien.

Son très faible diamètre permet même d'introduire le microcâble dans une première veine (veine "aller") puis par une anastomose vers une deuxième veine (veine "retour") en remontant dans celle-ci. On a en effet constaté la présence très fréquente d'anastomoses distales dans le réseau veineux coronarien, c'est-à-dire qu'il existe à l'extrémité de certaines veines un passage vers une autre veine, avec de ce fait possibilité de communication entre deux veines distinctes au niveau de l'anastomose, via leurs extrémités distales respectives. Il devient ainsi possible, avec une unique sonde, de stimuler concomitamment deux zones relativement distantes, car situées dans deux veines distinctes. Le double effet d'éloignement de ces deux zones et de multiplication des points de stimulation dans chacune des zones procure un effet particulièrement bénéfique pour la resynchronisation du fonctionnement du cœur.

Un autre avantage du faible diamètre de la partie active de la sonde est qu'il évite l'obstruction d'une partie du flux sanguin dans la veine, qui en-trainerait une déficience de l'irrigation du réseau veineux situé en aval de l'extrémité de la sonde.

La réduction du diamètre de la sonde n'est toutefois pas dépourvue d'inconvénients.

En effet, lorsque le diamètre de la sonde est notablement inférieur à celui de la veine, il est difficile de garantir le contact permanent des électrodes. La partie dénudée du microcâble, qui forme une électrode, peut ainsi se trouver dans une position intermédiaire, "flottante", au milieu de la veine, les points de contact entre le microcâble et la paroi de la veine se faisant sur des régions électriquement isolées.

Au surplus, même en cas de contact effectif entre les électrodes et la paroi de la veine, cette configuration n'est pas stable, du fait des battements permanents du cœur.

Ce phénomène est particulièrement marqué dans le cas des microcâbles passés par une anastomose : en effet, si les veines sont de faible diamètre dans la région de l'anastomose, typiquement moins de 1 French (0,33 mm), au-delà de l'anastomose elles peuvent rejoindre le sinus coronaire après avoir contourné le ventricule gauche, et dans ce cas leur diamètre augmente. Le microcâble très fin, qui a permis de passer l'anastomose, risque alors d'évoluer dans une région de diamètre relativement large, avec de ce fait une difficulté à établir un contact stable entre les électrodes et la paroi de la veine dans cette région.

L'un des problèmes de l'invention est ainsi de proposer une structure de microsonde permettant de garantir le contact permanent de l'ensemble des électrodes avec les veines coronaires, en fixant la position de la sonde afin de pérenniser l'efficacité de la stimulation.

Un autre problème est celui du risque de déplacement de la partie active de la sonde après que celle-ci a été implantée.

Le EP 2 455 131 A1 précité prévoit pour cela des moyens de retenue, par exemple sous forme d'un relief hélicoïdal formé sur l'extrémité du corps de sonde au voisinage de l'extrémité de celui-ci, près du débouché d'où émerge le microcâble. L'extrémité du corps de sonde présente ainsi un diamètre localement accru, de l'ordre de 7 French (2,33 mm) permettant le maintien mécanique du corps de sonde dans la veine.

Le FR 2 801 510 A1 (correspondant au US 6 549 812 B1) décrit une sonde d'un type comparable, également munie de moyens de retenue mécanique de la sonde dans la veine-cible.

Ce moyen de retenue présente cependant l'inconvénient d'être volumineux et d'oblitérer partiellement le passage du flux sanguin dans la veine. Au surplus, s'il permet une bonne retenue du corps de sonde, il ne protège pas la partie de la sonde introduite dans le réseau veineux profond, en l'espèce le microcâble télescopique, à l'encontre des déplacements provoqués par des mouvements amples du patient, par exemple lorsque celui-ci lève son bras : un tel mouvement tend à allonger la veine cave supérieure, avec un risque de traction locale exercée sur la sonde, traction qui est transmise jusqu'à la région distale implantée dans le réseau coronaire. Ces mouvements engendrés par le corps humain sont ainsi un facteur supplémentaire de déplacement des électrodes placées sur la zone de stimulation.

L'invention a pour objet de résoudre les problèmes évoqués ci-dessus.

Elle propose à cet effet une microsonde de détection/stimulation du type général divulgué par le FR 2 801 510 A1 précité, destinée à être implantée dans un vaisseau cible, notamment un vaisseau du réseau veineux coronarien pour la stimulation d'une cavité du cœur.

Une telle microsonde comporte une partie active distale formée par un microcâble de diamètre au plus égal à 2 French (0,66 mm) électriquement conducteur, ce microcâble étant revêtu d'une couche d'isolement comprenant, le long de ladite partie active et à distance de l'extrémité distale de la microsonde, une ou plusieurs zones dénudées formant électrode(s) de stimulation, destinées à venir en contact avec une paroi du vaisseau cible. La microsonde comprend une zone de stimulation à l'extrémité distale du microcâble, dans une région comprenant les électrodes, et une zone de rétention comprenant des moyens de retenue aptes à venir en appui contre la paroi du vaisseau cible.

De façon caractéristique de l'invention, cette microsonde comprend en outre une zone d'élongation, située en position proximale par rapport à la zone de rétention à une distance de celle-ci comprise entre 5 et 150 mm et définie par une région conformée de la microsonde apte à rendre celle-ci élastiquement déformable dans le sens longitudinal sous l'effet d'une sollicitation de traction/compression axiale exercée sur la microsonde dans sa région proximale par rapport à la zone d'élongation, la raideur en traction/compression axiale de la zone d'élongation étant inférieure à la raideur en traction/compression axiale des zones de rétention et de stimulation.

Ainsi, les sollicitations axiales exercées sur la partie proximale de la sonde entre la zone d'élongation et un connecteur monté à l'extrémité proximale de la sonde seront absorbées par une déformation de la zone d'élongation. Cette déformation de la zone d'élongation aura donc lieu sans déplacement des zones de rétention et de stimulation, par voie de conséquence sans modification corrélative du positionnement des électrodes.

Selon diverses caractéristiques subsidiaires avantageuses :
- la partie active distale est dépourvue de lumière centrale interne ;
- la zone de stimulation est définie par une première préforme courbée du microcâble à l'extrémité distale de celui-ci ;
- la longueur de la zone d'élongation est, à l'état libre de la microsonde, comprise entre 10 et 50 mm et ses dimensions hors tout en direction radiale sont, à l'état libre de la microsonde, comprises dans une enveloppe de diamètre apparent compris entre 5 et 20 mm ;
- la raideur en traction/compression axiale de la zone d'élongation est comprise entre 1 et 2,5 N/mm², et celle des zones de rétention et de stimulation est comprise entre 2,5 et 5 N/mm² ;
- la longueur de la zone de rétention est, à l'état libre de la microsonde, comprise entre 10 et 40 mm et ses dimensions hors tout en direction radiale sont, à l'état libre de la microsonde, comprises dans une enveloppe de diamètre apparent compris entre 10 et 50 mm.

Dans une première forme de réalisation, la zone d'élongation est définie par une deuxième préforme du microcâble. La zone de rétention et la zone de stimulation peuvent être des zones confondues, définies par une même région du microcâble. Le microcâble comprend avantageusement une couche de revêtement extérieur en polymère à mémoire de forme constituant de façon permanente les première et deuxième préformes.

La microsonde comprend également un microcathéter de diamètre au plus égal à 2 French (0,66 mm), duquel émerge la partie distale du microcâble, et la zone d'élongation est définie par une première préforme du microcathéter. La zone de rétention et la zone de stimulation peuvent être des zones distinctes, la zone de rétention étant située en position proximale par rapport à la zone de stimulation, et la zone de rétention étant définie par une deuxième préforme du microcathéter.

On va maintenant décrire un exemple de mise en œuvre de l'invention, en référence aux dessins annexés où les mêmes références numériques désignent d'une figure à l'autre des éléments identiques ou fonctionnellement semblables.
La Figure 1 illustre de façon générale le myocarde, avec les principales veines du réseau coronarien dans lequel a été introduite une sonde selon l'invention, destinée à la stimulation du ventricule gauche.
La Figure 2 illustre un exemple de configuration dans laquelle une électrode d'un microcâble se trouve localement dans une région de diamètre relativement important d'une veine du réseau coronaire.
La Figure 3 illustre un exemple d'une microsonde seIon l'invention, constituée d'un microcâble sur lequel ont été formées une zone d'élongation selon l'invention et une zone combinée de rétention et stimulation.
La Figure 4 illustre un mode de réalisation d'une microsonde selon l'invention, constituée d'un microcâble avec une zone combinée de rétention et stimulation, et une zone d'élongation formée dans une région d'un microcathéter logeant le microcâble.
La Figure 5 illustre un autre mode de réalisation d'une microsonde selon l'invention, combinant un microcâble où a été formée une zone de stimulation, et un microcathéter logeant ce microcâble et sur lequel ont été formées une zone de rétention et une zone d'élongation distinctes.
Les Figures 6 à 10 illustrent divers exemples de configurations de préformes utilisables pour les zones d'élongation, de rétention et/ou de stimulation de la microsonde selon l'invention.

On va maintenant décrire un exemple de réalisation du dispositif de l'invention.

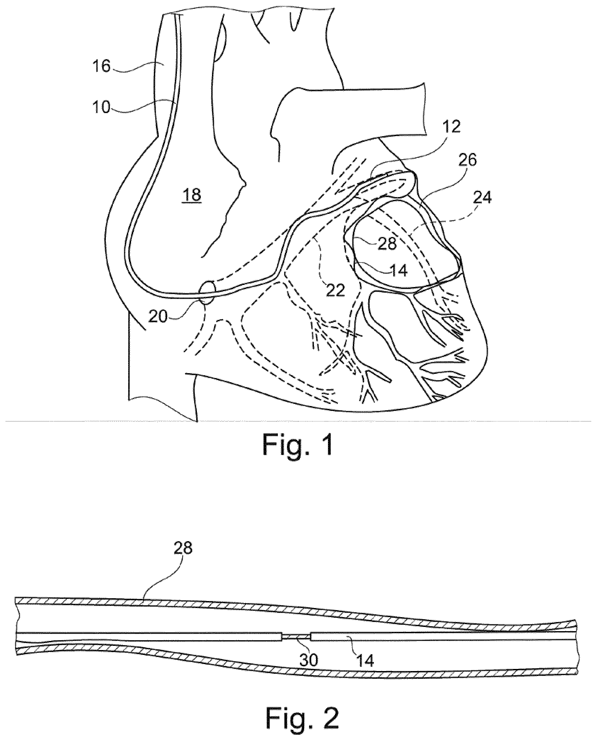

La Figure 1 illustre de façon générale le myocarde et les principaux vaisseaux du réseau coronarien, dans lequel on a introduit une sonde 10 afin de stimuler le ventricule gauche.

La partie distale de la sonde 10, à savoir la partie destinée à pénétrer dans les vaisseaux du réseau coronarien profond, comprend dans l'exemple illustré un microcathéter 12 constituant la partie distale du corps de sonde.

Le microcathéter 12 est apte à pénétrer dans le réseau profond, donc présente une partie distale de diamètre très réduit et, d'autre part, est destiné à être laissé en place après la pose de la microsonde, donc doit répondre à toutes les contraintes imposées à un accessoire présent à demeure dans le réseau veineux.

Ce microcathéter comprend une lumière centrale logeant un microcâble 14 de diamètre au plus égal à 2 French (0,66 mm), typiquement de l'ordre de 0,5 à 2 French (0,16 à 0,66 mm) portant dans sa partie distale, active, une pluralité d'électrodes de détection/stimulation destinées à stimuler le ventricule gauche à partir de plusieurs sites situés dans la veine coronaire.

Plus précisément, la sonde 10 est implantée par voie endocavitaire dans le réseau veineux coronarien via la veine cave supérieure 16, l'oreillette droite 18 et l'entrée du sinus coronaire veineux 20. Le réseau veineux coronarien se développe ensuite en plusieurs branches à partir de la grande veine coronaire 22, ces branches comprenant les veines postérolatérale 24, latérale 26, et antérolatérale 28.

Si nécessaire, la microsonde (c'est-à-dire le microcâble ou l'ensemble microcâble/microcathéter) peut atteindre et traverser une anastomose. Une anastomose est un passage existant, à l'extrémité de certaines veines du réseau coronaire, vers une autre veine, avec de ce fait possibilité de communication entre deux veines distinctes au niveau de l'anastomose, via leurs extrémités distales respectives. Dans ce cas, la microsonde est canulée dans une première veine (veine "aller") puis par l'anastomose vers une deuxième veine (veine "retour") en remontant dans celle-ci, ce qui permet de placer l'extrémité active du microcâble dans des veines de très faible diamètre, non exploitées à ce jour du fait de la taille excessive des sondes coronaires permanentes conventionnelles. Grâce à cette configuration, il est possible non seulement de stimuler le ventricule gauche à partir de points situés profondément dans une veine du réseau coronaire, mais également, via l'anastomose, dans des régions proximales de veines dans lesquelles il aurait été difficile de stabiliser ou fixer des sondes conventionnelles de stimulation du ventricule gauche, du fait du grand diamètre de l'embouchure de ces veines.

La Figure 2 illustre le microcathéter 12, avec une zone dénudée 30 formant électrode, s'étendant dans une veine 28 dans une configuration où l'électrode 30 n'est pas en contact avec la paroi de cette veine. En effet, l'une des difficultés rencontrées avec les microcâbles, dont le diamètre est très faible pour pouvoir pénétrer le réseau veineux profond et même si nécessaire traverser une anastomose, peut être un inconvénient dans une région où le vaisseau est relativement large : dans ce cas, le diamètre réduit du microcâble nuit à la stabilité de la position des zones d'électrodes, car le câble vient "flotter" à l'intérieur de la veine, avec une incidence notable sur l'obtention d'un bon contact entre électrode et paroi, et donc sur l'efficacité de la stimulation.

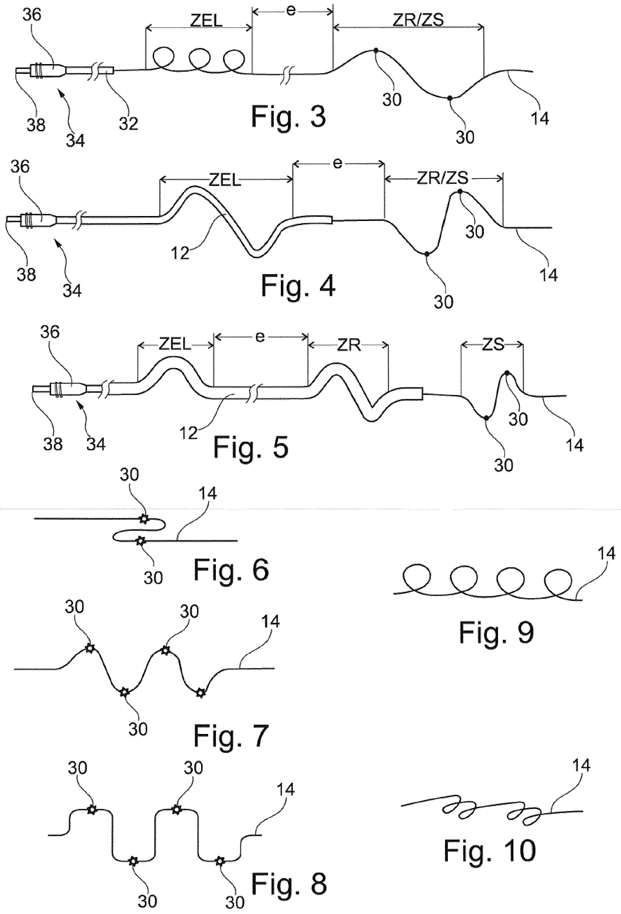

La Figure 3 illustre un exemple qui ne fait pas partie de l'objet revendiqué, appliquée à une configuration où la microsonde est essentiellement constituée par la partie distale du microcâble 14, c'est-à-dire dans une configuration ne faisant pas usage d'un microcathéter (le corps de sonde creux n'allant pas au-delà de la grande veine coronaire 22). La microsonde est alors constituée du seul microcâble 14, qui dans sa partie distale émerge du corps de sonde 32. Côté proximal, le microcâble 14 court sur toute la longueur du corps de sonde 32 jusqu'à une fiche de connecteur 34 dont le corps 36 est solidaire du corps de sonde 32 et la broche 38 est liée mécaniquement et électriquement au microcâble 14.

Le microcâble 14 présente un diamètre au plus égal à 2 French (0,66 mm), typiquement de l'ordre de 0,5 à 2 French (0,16 à 0,66 mm), et porte une pluralité d'électrodes de détection/stimulation 30, avec une préforme bi-ou tridimensionnelle, par exemple en forme d'ondulation, pour favoriser le contact des électrodes avec les parois des veines, et par là même la performance électrique.

Un tel microcâble est par exemple décrit dans le EP 2 455 131 A1 précité, auquel on pourra se référer pour de plus amples détails, ainsi que sur la manière dont il peut être implanté dans le réseau coronaire.

Le microcâble 14 est pourvu d'un revêtement isolant sur toute sa longueur, à l'exception de zones ponctuellement dénudées servant à constituer les électrodes de détection/stimulation, par exemple les zones ponctuellement dénudées 30 situées au sommet de chacune des deux ondulations de la préforme illustrée.

En ce qui concerne la structure du microcâble 14, le cœur de celui-ci est avantageusement une structure multifilaire dans laquelle chaque brin est de préférence constitué de nitinol (alliage NiTi) ou de MP35N-LT (35 % Ni, 35 % Co, 20 % Cr et 10 % Mo), matériaux dont l'avantage essentiel est leur extrême endurance en fatigue, avec enrobage par une gaine en platine iridié ou en tantale (pour la radioopacité et la biostabilité). Une telle structure permet d'optimiser la réponse aux exigences de résistance à la corrosion au niveau des électrodes et d'endurance à l'encontre des mouvements cardiaques. Ces microcâbles sont disponibles par exemple auprès de la société Fort Wayne Metals Inc., Fort Wayne, USA.

Le câble de cœur est revêtu d'une fine couche d'isolement, de l'ordre de 25 µm d'épaisseur, par exemple par co-extrusion sur le conducteur ou par échauffement d'un tube thermorétractable. L'isolant peut être :
- une fine couche de parylène (par exemple de type C) : dans ce cas, des fenêtres plus ou moins complexes sont aménagées le long du microcâble, par exemple par ablation plasma, pour former les électrodes 30 ; pour améliorer les performances électriques, ces zones peuvent être en outre revêtues par exemple de nitrure de titane ;
- un tube polyuréthanne interrompu aux endroits des électrodes 30 ;
- une ou plusieurs couches constituées de tubes en PET (polyéthylène téréphtalate), polymère fluoré, PMMA (polyméthacrylate de méthyle), PEEK (polyétheréthercétone), polyimide ou autre matériau similaire approprié.

Un avantage, particulièrement significatif, de cette structure résulte du caractère très souple et flottant (*floppy*) du microcâble, qui lui confère une excellente atraumaticité ; un tel microcâble agresse peu les tissus et préserve donc les cellules à proximité immédiate des électrodes.

Le point de départ de l'invention consiste à distinguer fonctionnellement trois zones, à savoir :
- une zone de stimulation ZS, qui est la zone supportant les électrodes ;
- une ou plusieurs zones de rétention ZR, dont le rôle est d'assurer le maintien en place de la sonde dans le vaisseau ; et
- une zone d'élongation ZEL capable d'absorber les mouvements relatifs engendrés par le corps humain ; son rôle est celui d'une zone tampon évitant tout déplacement des zones de stimulation et de rétention après que la sonde a été implantée.

Les zones de rétention ZR et de stimulation ZS peuvent être confondues en une zone combinée de rétention et stimulation ZR/ZS (comme dans le cas des Figures 3 et 4), la rétention de la partie active de la sonde dans le vaisseau résultant alors de la préforme donnée à la partie active ou zone de stimulation ZS.

Dans l'exemple de la Figure 3, la zone d'élongation ZEL est constituée par une conformation donnée au microcâble 14 dans une région située en position proximale par rapport à la zone combinée de rétention et stimulation ZR/ZS, distincte de cette dernière et éloignée de celle-ci d'une distance e.

Dans l'exemple illustré, la conformation de la zone d'élongation ZEL est une conformation hélicoïdale, et le dimensionnement de cette zone et sa position par rapport au reste de la microsonde sont choisis de manière que la zone d'élongation ZEL se situe dans une région du réseau coronaire de diamètre relativement large, par exemple à proximité du sinus coronaire 20, ou même en dehors du réseau coronaire, par exemple dans la veine cave supérieure 16 ou selon le cas toute autre veine située entre le connecteur 34 à l'extrémité proximale et la zone de rétention et stimulation ZR/ZS à l'extrémité distale.

L'intervalle e entre la zone d'élongation ZEL et la zone de rétention et stimulation ZR/ZS peut ainsi être compris entre 5 et 150 mm.

La zone d'élongation présente une longueur typique de 10 à 50 mm, et ses dimensions hors tout en direction radiale sont comprises dans une enveloppe de diamètre apparent compris entre 5 et 20 mm (ici et dans la suite, toutes les dimensions sont données à l'état libre de la microsonde, avant implantation).

En ce qui concerne la zone combinée de rétention et stimulation ZR/ZS, celle-ci présente une longueur typique de 10 à 40 mm, avec des dimensions hors tout de la préforme en direction radiale comprises dans une enveloppe de diamètre apparent compris entre 10 et 50 mm (ici encore, à l'état libre de la microsonde).

De façon caractéristique de l'invention, dans ce mode de réalisation et dans les suivants, la raideur en traction/compression axiale de la zone d'élongation ZEL est inférieure à celle de la zone de rétention et stimulation ZR/ZS.

En d'autres termes, l'effort axial nécessaire pour déformer la zone d'élongation est inférieur à l'effort exercé par la zone de rétention, ce qui permet d'assurer qu'il n'y ait pas de déplacement des électrodes de la zone de stimulation. Ainsi, même si le patient bouge et exécute des mouvements amples (levée du bras, etc.), les sollicitations exercées sur la partie proximale de la sonde (entre la zone d'élongation ZEL et le connecteur 34) seront absorbées par une déformation de la zone d'élongation ZEL, sans déplacement des zones de rétention et stimulation ZR/ZS donc sans effet sur le positionnement des électrodes.

La raideur en traction/compression axiale de la zone d'élongation ZEL est typiquement comprise entre 1 et 2,5 N/mm², pour une raideur en traction/compression axiale des zones de rétention et stimulation ZR/ZS comprise entre 2,5 et 5 N/mm. Ces valeurs permettent de réaliser une sonde conforme aux exigences de la norme EN 45502-2-1, qui définissent un effort de traction maximum admissible de 5 N sur les sondes, comparable à une situation d'explantation de sonde.

La Figure 4 illustre un mode de réalisation de l'invention, dans lequel le corps de sonde est terminé à sa partie distale par un microcathéter 12 duquel émerge le microcâble 14.

On dispose ainsi à l'intérieur du réseau coronaire d'un ensemble télescopique microcathéter/microcâble, avec possibilité de déplacement axial relatif entre le microcathéter 12 et le microcâble 14 permettant au chirurgien d'ajuster au moment de l'implantation la longueur émergente de la partie distale du microcâble.

Le microcathéter 12 (qui est destiné à être laissé en place après la pose de la microsonde) présente un diamètre très réduit, typiquement inférieur à 2 French (0,33 mm) pour pouvoir pénétrer dans le réseau coronaire profond. Ce microcathéter est avantageusement réalisé par une technique d'extrusion à débit variable de deux matières avec, à l'intérieur, un tube définissant une lumière interne, par exemple un tube en PTFE, recevant par extrusion un revêtement d'un matériau choisi pour ses propriétés de souplesse, de résistance mécanique, de résistance à l'abrasion, de capacité à être extrudé et de biocompatibilité. Le matériau du revêtement peut être par exemple un polyuréthanne utilisable pour des applications d'implantation à long terme, ou bien un copolymère de polyuréthannes, un copolymère de polyuréthanne et de silicium, un polyéther bloc amide (par exemple de type *Pebax*), ou encore un polyamide. Ce matériau peut être chargé d'un opacifiant aux rayons X (BASO₄ ou TiO₂ par exemple) permettant de visualiser plus aisément le microcathéter sous amplificateur de brillance au moment de l'intervention. Un revêtement biocompatible améliorant le glissement contre les parois des vaisseaux, par exemple un revêtement hydrophile de type polyvinylpyrrolidone (PVP) ou silicone, peut être prévu à la surface externe du microcathéter.

Dans le mode de réalisation illustré sur la Figure 4, la microsonde, qui est ici formée par l'association du microcâble 14 et du microcathéter 12, comporte une zone d'élongation ZEL formée une préforme du microcathéter 12, par exemple une préforme avec des ondulations, préalablement obtenue grâce aux propriétés de mémoire de forme du polyuréthanne du microcathéter.

Le microcâble 14 comporte en partie distale une zone combinée de rétention et stimulation ZR/ZS comparable à celle de l'exemple de la Figure 3.

Les différentes dimensions données pour ce mode de réalisation sont applicables également à celui de la Figure 4.

En dehors des zones de rétention et stimulation ZR/ZS, le microcâble présente une forme rectiligne, se contentant d'épouser les courbures du microcathéter 12 notamment dans la zone d'élongation ZEL.

La Figure 5 illustre un autre mode de réalisation, qui est une variante du précédent dans lequel les zones de rétention ZR et de stimulation ZS sont distinctes. La zone de rétention ZR est formée sur le microcathéter 12, tandis que la zone de stimulation ZS est formée sur le microcâble.

La longueur de la zone de rétention ZR est typiquement comprise entre 10 et 40 mm, et celle de la zone de stimulation ZS entre 20 et 60 mm. Les autres dimensions peuvent être les mêmes que celles données dans l'exemple de la Figure 3.

Ici encore, la zone de rétention ZR du microcathéter 12 présente une raideur en traction/compression axiale supérieure à celle de la zone d'élongation ZEL, de manière à ce que celle-ci puisse préserver le placement de la zone de rétention ZR en absorbant les sollicitations axiales subies par la partie proximale de la sonde (entre la zone d'élongation ZEL et le connecteur 34).

Les Figures 6 à 10 illustrent diverses configurations de préformes bi- ou tridimensionnelles pouvant être utilisées pour les zones d'élongation ZEL, de rétention ZR et/ou de stimulation ZS.

Si ces préformes sont utilisées pour la zone de stimulation ZS, les électrodes de stimulation, symbolisées par les étoiles 30, sont placées judicieusement afin de garantir le contact avec les tissus du vaisseau. Pour de bonnes performances électriques, il est en effet important que les électrodes de stimulation 30 soient les plus proches possible des tissus stimulables.

Les préformes peuvent jouer un double rôle de rétention et de stimulation suivant la configuration choisie, d'une part en créant un effort de contact pour les électrodes sur la paroi veineuse, et d'autre part en appliquant une force de rétention suffisante pour bloquer le microcâble 14 dans le vaisseau coronaire.

Les différentes configurations illustrées ont toutes pour but d'augmenter artificiellement le diamètre du système implantable (microcâble seul ou ensemble microcathéter/microcâble). On peut avoir ainsi une configuration :
- en accordéon (Figure 6) ;
- avec une succession d'ondulations (Figure 7), cette configuration correspondant à celle des Figures 3, 4 et 5 pour les zones de rétention et/ou de stimulation ZR/ZS, et à celle de la Figure 5 également pour la zone d'élongation ZEL ;
- en créneaux successifs (Figure 8) ;
- en hélice (Figure 9), cette configuration correspondant à celle de la Figure 3 pour la zone d'élongation ZEL. Cette configuration peut être également appliquée de façon avantageuse à la zone de stimulation ZS, car du fait de la forme hélicoïdale qui épouse la paroi du vaisseau pratiquement en tous points, les électrodes peuvent se trouver à n'importe quel endroit et l'on sera certain qu'elles seront à peu près toujours en contact avec cette paroi quelle que soit leur localisation ;
- en forme de ressorts radiaux (Figure 10).

Dans toutes ces conformations, qu'elles soient bi- ou tridimensionnelles, grâce à l'élasticité du microcâble les préformes sont suffisamment déformables sous contrainte radiale lors du passage dans les veines du réseau coronaire et ne créent donc pas d'obstacle au moment de l'implantation.

## Revendications

1. Une microsonde de détection/stimulation destinée à être implantée dans un vaisseau cible, notamment un vaisseau du réseau veineux coronarien (22-28) pour la stimulation d'une cavité du coeur,
cette microsonde comportant une partie active distale formée par un microcâble (14) de diamètre au plus égal à 2 French (0,66 mm), ce microcâble étant électriquement conducteur et revêtu d'une couche d'isolement comprenant, le long de ladite partie active et à distance de l'extrémité distale de la microsonde, une ou plusieurs zones dénudées (30) formant électrode(s) de stimulation, destinées à venir en contact avec une paroi du vaisseau cible,
la microsonde comprenant :
- une zone de stimulation (ZS) à l'extrémité distale du microcâble, dans une région comprenant les électrodes (30) ; et
- une zone de rétention (ZR), comprenant des moyens de retenue aptes à venir en appui contre la paroi du vaisseau cible,
cette microsonde comprend en outre :
- une zone d'élongation (ZEL), située en position proximale par rapport à la zone de rétention à une distance de celle-ci comprise entre 5 et 150 mm et définie par une région conformée de la microsonde apte à rendre celle-ci élastiquement déformable dans le sens longitudinal sous l'effet d'une sollicitation de traction/compression axiale exercée sur la microsonde dans sa région proximale par rapport à la zone d'élongation,
la raideur en traction/compression axiale de la zone d'élongation étant inférieure à la raideur en traction/compression axiale des zones de rétention et de stimulation ;
la microsonde configurée de manière à ce que les sollicitations axiales exercées sur la partie proximale de la sonde entre la zone d'élongation et un connecteur monté à l'extrémité proximale de la sonde seront absorbées par une déformation de la zone d'élongation;
la microsonde configurée de manière à ce que cette déformation de la zone d'élongation a lieu sans déplacement des zones de rétention et de stimulation, par voie de conséquence sans modification corrélative du positionnement des électrodes;
et
- la microsonde étant **caractérisée en ce qu'**elle comprend également un microcathéter (12) de diamètre au plus égal à 2 French (0,66 mm), duquel émerge la partie distale du microcâble (14), et
- la zone d'élongation (ZEL) est définie par une première préforme du microcathéter.

2. La microsonde de la revendication 1, dans laquelle la partie active distale est dépourvue de lumière centrale interne.

3. La microsonde de la revendication 1, dans laquelle la zone de stimulation (ZS) est définie par une première préforme courbée du microcâble à l'extrémité distale de celui-ci.

4. La microsonde de la revendication 1, dans laquelle la longueur de la zone d'élongation (ZEL) est comprise, à l'état libre de la microsonde, entre 10 et 50 mm.

5. La microsonde de la revendication 1, dans laquelle les dimensions hors tout en direction radiale de la zone d'élongation (ZEL) sont comprises, à l'état libre de la microsonde, dans une enveloppe de diamètre apparent compris entre 5 et 20 mm.

6. La microsonde de la revendication 1, dans laquelle la raideur en traction/compression axiale de la zone d'élongation (ZEL) est comprise entre 1 et 2,5 N/mm<2> .

7. La microsonde de la revendication 1, dans laquelle la raideur en traction/compression axiale des zones de rétention (ZR) et de stimulation (ZS) est comprise entre 2,5 et 5 N/mm<2> .

8. La microsonde de la revendication 1, dans laquelle la longueur de la zone de rétention (ZR) est comprise, à l'état libre de la microsonde, entre 10 et 40 mm.

9. La microsonde de la revendication 1, dans laquelle les dimensions hors tout en direction radiale de la zone de rétention (ZR) sont comprises, à l'état libre de la microsonde, dans une enveloppe de diamètre apparent compris entre 10 et 50 mm.

10. La microsonde de la revendication 1, dans laquelle la zone d'élongation (ZEL) est définie par une deuxième préforme du microcâble.

11. La microsonde de la revendication 10, dans laquelle la zone de rétention et la zone de stimulation sont des zones confondues (ZR/ZS), définies par une même région du microcâble.

12. La microsonde de la revendication 10, dans laquelle le microcâble (14) comprend une couche de revêtement extérieur en polymère à mémoire de forme constituant de façon permanente lesdites première et deuxième préformes.

13. La microsonde de la revendication 1, dans laquelle :
- la zone de rétention (ZR) et la zone de stimulation (ZS) sont des zones distinctes, la zone de rétention étant située en position proximale par rapport à la zone de stimulation, et
- la zone de rétention (ZR) est définie par une deuxième préforme du microcathéter.

## Patentansprüche

1. Ein Sensor-/Stimulationsmikroleiter zur Implantation in ein Zielgefäß, insbesondere ein Gefäß des Herzkranznetzwerks (22-28), zur Stimulation einer Herzkammer,
wobei dieser Mikroleiter einen distalen aktiven Teil aufweist, der von einem Mikrokabel (14) mit einem Durchmesser von höchstens 2 French (0,66 mm) gebildet wird, wobei dieses Mikrokabel elektrisch leitend und mit einer Isolierschicht überzogen ist, die entlang des aktiven Teils und in einem Abstand vom distalen Ende des Mikroleiters eine oder mehrere freigelegte Zonen (30) umfasst, die Stimulationselektrode(n) bilden und dazu bestimmt sind, mit einer Wand des Zielgefäßes in Kontakt zu kommen, wobei der Mikroleiter umfasst:
- eine Stimulationszone (ZS) am distalen Ende des Mikrokabels in einem Bereich, der die Elektroden (30) umfasst; und
- eine Rückhaltezone (ZR), die Rückhaltemittel umfasst, die geeignet sind, an der Wand des Zielgefäßes anzuliegen,
wobei dieser Mikroleiter außerdem umfasst:
- eine Dehnungszone (ZEL), die sich in proximaler Position in Bezug auf die Retentionszone in einem Abstand von dieser zwischen 5 und 150 mm befindet und durch einen geformten Bereich des Mikroleiters definiert ist, der geeignet ist, diese unter der Wirkung einer axialen Zug-/Druckbeanspruchung, die auf den Mikroleiter in ihrem proximalen Bereich in Bezug auf die Dehnungszone ausgeübt wird, in Längsrichtung elastisch verformbar zu machen,
wobei die axiale Zug-/Drucksteifigkeit des Dehnungsbereichs geringer ist als die axiale Zug-/Drucksteifigkeit der Retentions- und Stimulationsbereiche; der Mikroleiter so konfiguriert ist, dass die axialen Belastungen, die auf den proximalen Teil der Sonde zwischen der Verlängerungszone und einem am proximalen Ende der Sonde angebrachten Verbinder ausgeübt werden, durch eine Verformung der Verlängerungszone absorbiert werden;
der Mikroleiter, der so konfiguriert ist, dass diese Verformung der Dehnungszone ohne Verschiebung der Retentions- und Stimulationszonen stattfindet, folglich ohne korrelative Änderung der Positionierung der Elektroden;
und
- wobei der Mikroleiter **dadurch gekennzeichnet ist, dass** sie auch einen Mikrokatheter (12) mit einem Durchmesser von höchstens 2 French (0,66 mm) umfasst, aus dem der distale Teil des Mikrokabels (14) herausragt, und
- die Dehnungszone (ZEL) durch eine erste Vorform des Mikrokatheters definiert ist.

2. Der Mikroleiter nach Anspruch 1, wobei der distale aktive Teil kein inneres zentrales Lumen aufweist.

3. Der Mikroleiter nach Anspruch 1, wobei die Stimulationszone (ZS) durch eine erste gebogene Vorform des Mikrokabels an dessen distalem Ende definiert ist.

4. Der Mikroleiter nach Anspruch 1, wobei die Länge der Dehnungszone (ZEL) im freien Zustand der Mikroleiter zwischen 10 und 50 mm beträgt.

5. Der Mikroleiter nach Anspruch 1, wobei die Gesamtabmessungen in radialer Richtung der Dehnungszone (ZEL) im freien Zustand der Mikroleiter innerhalb einer Hülle mit einem scheinbaren Durchmesser zwischen 5 und 20 mm liegen.

6. Der Mikroleiter nach Anspruch 1, wobei die axiale Zug-/Drucksteifigkeit der Dehnungszone (ZEL) zwischen 1 und 2,5 N/mm<2> liegt.

7. Der Mikroleiter nach Anspruch 1, wobei die axiale Zug-/Drucksteifigkeit der Retentions- (ZR) und Stimulationszonen (ZS) zwischen 2,5 und 5 N/mm<2> liegt.

8. Der Mikroleiter nach Anspruch 1, wobei die Länge der Retentionszone (ZR) im freien Zustand der Mikroleiter zwischen 10 und 40 mm beträgt.

9. Der Mikroleiter nach Anspruch 1, wobei die Gesamtabmessungen in radialer Richtung der Retentionszone (ZR) im freien Zustand des Mikroleiters innerhalb einer Hülle mit einem scheinbaren Durchmesser zwischen 10 und 50 mm liegen.

10. Der Mikroleiter nach Anspruch 1, wobei die Dehnungszone (ZEL) durch eine zweite Vorform des Mikrokabels definiert ist.

11. Der Mikroleiter nach Anspruch 10, wobei die Retentionszone und die Stimulationszone zusammenfallende Bereiche (ZR/ZS) sind, die durch denselben Bereich des Mikrokabels definiert werden.

12. Der Mikroleiter nach Anspruch 10, wobei das Mikrokabel (14) eine äußere Deckschicht aus einem Formgedächtnispolymer umfasst, die dauerhaft die erste und die zweite Vorform bildet.

13. Der Mikroleiter nach Anspruch 1, wobei:
- der Retentionsbereich (ZR) und der Stimulationsbereich (ZS) getrennte Bereiche sind, wobei sich der Retentionsbereich proximal zum Stimulationsbereich befindet,
und
- der Retentionsbereich (ZR) durch eine zweite Vorform des Mikrokatheters definiert ist.

## Claims

1. A sensing/stimulation microlead for implantation into a target vessel, in particular a vessel of the coronary network (22-28), for stimulation of a ventricle, said microlead having a distal active portion formed by a microcable (14) having a diameter of at most 2 French (0.66 mm), said microcable being electrically conductive and being coated with an insulating layer comprising, along the active portion and at a distance from the distal end of the microlead, one or more exposed zones (30) forming stimulation electrode(s) and intended to come into contact with a wall of the target vessel, said microlead comprising:
- a stimulation zone (ZS) at the distal end of the microcable in a region comprising the electrodes (30); and
- a restraint zone (ZR) comprising restraining means suitable for resting against the wall of the target vessel,
said microlead further comprising:
- an elongation zone (ZEL) located in proximal position with respect to the retention zone at a distance therefrom of between 5 and 150 mm and defined by a shaped portion of the microlead adapted to render it elastically deformable longitudinally under the effect of an axial tensile/compressive stress exerted on the microlead in its proximal portion with respect to the elongation zone, wherein the axial tensile/compressive stiffness of the expansion region is less than the axial tensile/compressive stiffness of the retention and stimulation regions;
the microlead is configured such that the axial loads applied to the proximal part of the probe between the extension zone and a connector attached to the proximal end of the probe are absorbed by a deformation of the extension zone;
the microlead configured so that this deformation of the strain zone takes place without displacement of the retention and stimulation zones, consequently without correlative change in the positioning of the electrodes; and
- the microlead being **characterised in that** it also comprises a microcatheter (12) having a diameter of at most 2 French (0.66 mm) from which the distal part of the microcable (14) protrudes, and
- the elongation zone (ZEL) is defined by a first preform of the microcatheter.

2. The microlead of claim 1, wherein the distal active portion does not have an inner central lumen.

3. The microlead of claim 1, wherein the stimulation zone (ZS) is defined by a first curved preform of the microcable at its distal end.

4. The microlead according to claim 1, wherein the length of the elongation zone (ZEL) in the free state of the microlead is between 10 and 50 mm.

5. The microlead of claim 1, wherein the overall dimensions in the radial direction of the strain zone (ZEL) in the free state of the microlead are within an envelope having an apparent diameter between 5 and 20 mm.

6. The microlead of claim 1, wherein the axial tensile/compressive stiffness of the strain zone (ZEL) is between 1 and 2.5 N/mm<2>.

7. The microlead of claim 1, wherein the axial tensile/compressive stiffness of the retention (ZR) and stimulation (ZS) zones is between 2.5 and 5 N/mm<2>.

8. The microlead according to claim 1, wherein the length of the retention zone (ZR) in the free state of the microlead is between 10 and 40 mm.

9. The microlead of claim 1, wherein the overall dimensions in the radial direction of the retention zone (ZR) in the free state of the microlead are within an envelope having an apparent diameter between 10 and 50 mm.

10. The microlead of claim 1, wherein the strain zone (ZEL) is defined by a second preform of the microcable.

11. The microlead of claim 10, wherein the retention zone and the stimulation zone are coincident regions (ZR/ZS) defined by the same region of the microcable.

12. The microlead of claim 10, wherein the microcable (14) comprises an outer cover layer of a shape memory polymer that permanently forms the first and second preforms.

13. The microlead of claim 1, wherein:
- the retention area (ZR) and the stimulation area (ZS) are separate areas, the retention area being proximal to the stimulation area,
and
- the retention area (ZR) is defined by a second preform of the microcatheter.
